(19) Europäisches Patentamt
European Patent Office
Office européen des brevets

(11) **EP 1 285 625 A2**

(12) **EUROPÄISCHE PATENTANMELDUNG**

(43) Veröffentlichungstag:
26.02.2003 Patentblatt 2003/09

(51) Int Cl.$^7$: **A61B 5/00**, C12P 7/56

(21) Anmeldenummer: 02017870.3

(22) Anmeldetag: 08.08.2002

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **10.08.2001 DE 10138289**

(71) Anmelder: **Pfitzenmeier, Werner
68723 Schwetzingen (DE)**

(72) Erfinder: **Pfitzenmeier, Werner
68723 Schwetzingen (DE)**

(74) Vertreter: **Schmitt, Meinrad, Dipl.-Ing.
Reble, Klose & Schmitt
Patente & Marken
Postfach 12 15 19
68066 Mannheim (DE)**

(54) **Verfahren zur Trainingssteuerung**

(57) Die Erfindung bezieht sich auf ein Verfahren zur Trainingssteuerung, insbesondere zur Erlangung eines vorgebbaren Fettabbaus und / oder Muskelaufbaus und/oder Steigerung der Ausdauer oder Leistung einer oder eines Trainierenden. Der Erfindung liegt die Aufgabe zugrunde, das Verfahren dahingehend auszubilden, dass einem Trainierenden die für ein erfolgreiches Training erforderlichen Daten oder Werte bereitgestellt werden. Hierzu wird vorgeschlagen, dass der Laktatgehalt des Blutes der oder des Trainierenden erfasst wird und unter Berücksichtigung einer vorgegebenen oder vorgebbaren aeroben / anaeroben Schwelle, vorzugsweise zwischen 2,3 - 6,8 mmol Laktat/l Blut, insbesondere in der Grössenordnung von 4 mmol Laktat /l Blut, das Trainingsziel vorgegeben wird.

EP 1 285 625 A2

**Beschreibung**

Die Erfindung bezieht sich auf ein Verfahren zur Trainingssteuerung unter Berücksichtigung einer vorgebbaren Schwelle des im menschlichen Blut enthaltenen Laktats.

In Sport- oder Fitness-Studios stehen einem Trainierenden heute die unterschiedlichsten Geräte zur Verfügung, welche je nach Trainingsziel zu einer Leistungssteigerung, zum Fettabbau, zur Erhöhung der Ausdauer oder für einen gezielten Muskelaufbau dienen. Für eine zielgerichtetes Training, sei es im Hinblick auf eine Gewichtsreduzierung bzw. einen Fettabbau oder einen Aufbau der Muskulatur, ist jedoch ein erheblicher personeller Aufwand für die Betreuung und Beratung des Trainierenden und die Auswertung der aufgrund des Trainings erzielten Werte erforderlich.

Es ist bekannt, dass nach Art, Umfang, Dauer usw. des Trainings und der dabei erfolgten Belastung des menschlichen Organismus die Laktatwerte im Blut infolge unterschiedlicher Verbrennung der im Organismus gespeicherten Energieträger sich verändern. So wird bei anaerober Verbrennung, also ohne Sauerstoffzufuhr, dem Organismus Energie unter Bildung von Laktat (La) bereit gestellt, während bei aerober Verbrennung mit Sauerstoffzufuhr vergleichsweise langsam eine grössere Energiemenge frei gesetzt wird. Insoweit ist ferner das Körpergewicht des Menschen bzw. dessen Blutmenge zu beachten, wobei grundsätzlich die Beziehung gilt, dass 1 mmol La/1 l Blut zumindest näherungsweise 0,75 mmol La/kg Körpergewicht entspricht, also:

$$1 \text{ mmol La} / 1 \text{ l Blut} = 0,75 \text{ mmol La} / \text{kg Körpergewicht}$$

Unter der Voraussetzung von beispielsweise 10 mmol La / 1 l Blut und einem Gesamtkörpergewicht von 70 kg gilt beispielsweise folgende Bedingung:

$$\text{gebildetes Laktat} = 10 \times 0,75 \times 70 = 525 \text{ mmol}$$

Der Erfindung liegt die Aufgabe zugrunde, ein Verfahren zur Trainingssteuerung vorzuschlagen, mit welchem in einfacher Weise und zielgerichtet dem Trainierenden die für ein erfolgreiches Training erforderlichen Daten und / oder Werte bereit zu stellen. Das Verfahren soll ferner problemlos auf die individuellen Bedürfnisse und Anforderungen ausrichtbar sein und in zuverlässiger Weise die Verarbeitung von spezifischen Daten des Trainierenden ermöglichen. Ferner soll das Verfahren in einfacher Weise mit einem Software-Programm realisierbar sein, wobei eine Anpassung und Berücksichtigung variabler, insbesondere vom jeweiligen Trainierenden abhängiger Parameter in einfacher Weise durchführbar sein soll.

Die Lösung dieser Aufgabe erfolgt gemäss den im Patentanspruch 1 angegebenen Merkmalen. In den Unteransprüchen sind besondere Ausgestaltungen und Weiterbildungen angegeben.

Das erfindungsgemässe Verfahren ermöglicht in vorteilhafter Weise die Trainingssteuerung unter Berücksichtigung der vorgebbaren aeroben / anaeroben Schwelle, insbesondere in der Grössenordnung von 4 mmol La / l Blut. Mittels eines geeigneten Gerätes wird der Laktatgehalt des Blutes gemessen und in einen Rechner eingegeben, wobei mittels des erfindungsgemässen Software-Programms die nachfolgenden Berechnungen und / oder Bewertungen durchgeführt werden. Das Software-Programm umfasst die Fettstoffwechsel-Ausdauer-Trainings-Steuerung, nachfolgend kurz FAT'S bezeichnet unter Berücksichtigung der gemessenen bzw. vorgegebenen aeroben /anaeroben Schwelle von insbesondere 4 mmol La / l Blut.

Mit der Bestimmung der Laktatschwelle wird die maximale Belastungsintensität ermittelt, bei welcher die Laktatdiffusion aus der Muskulatur in das Blut einerseits und die Laktatelimination aus dem Blut andererseits bei einer länger andauerden Belastung gerade noch im Gleichgewicht stehen. Die Laktatschwelle kann grundsätzlich bei einer Belastungsintensität bestimmt werden, wobei beispielsweise in einem Stufentest 4 mmol Laktat pro Liter Blut erreicht werden, wobei diese Laktatschwelle auch als anaerobe Schwelle bezeichnet wird. Darüber hinaus wurde erkannt, dass die Laktatschwelle individuell deutlich variieren kann, wobei Werte im Bereich von 2,3 bis 6,8 mmol Laktat pro Liter Blut festgestellt werden konnten. So wurde bei Ausdauer trainierten Personen festgestellt, daß Belastungen mit der Intensität der anaeroben Schwelle von 4 mmol Laktat / Liter Blut aufgrund einer unerwünschten Laktatakkumulation abgebrochen werden mußte. Daher erfolgt in einer besonderen Ausgestaltung der Erfindung die Bestimmung der individuellen anaeroben Laktatschwelle, nachfolgend kurz IAS genannt. Hierzu wird in einer ersten Ausgestaltung zu einem als Basislaktat bezeichneten Wert, welcher zu Beginn des Anstiegs der Laktatleistungskurve erfaßt wird, der Wert von 1,5 mmol Laktat pro Liter Blut addiert und hieraus die Belastungsintensität bestimmt. In einer bevorzugten Ausgestaltung wird die individuelle anaerobe Laktatschwelle in der nachfolgend erläuterten Weise bestimmt.

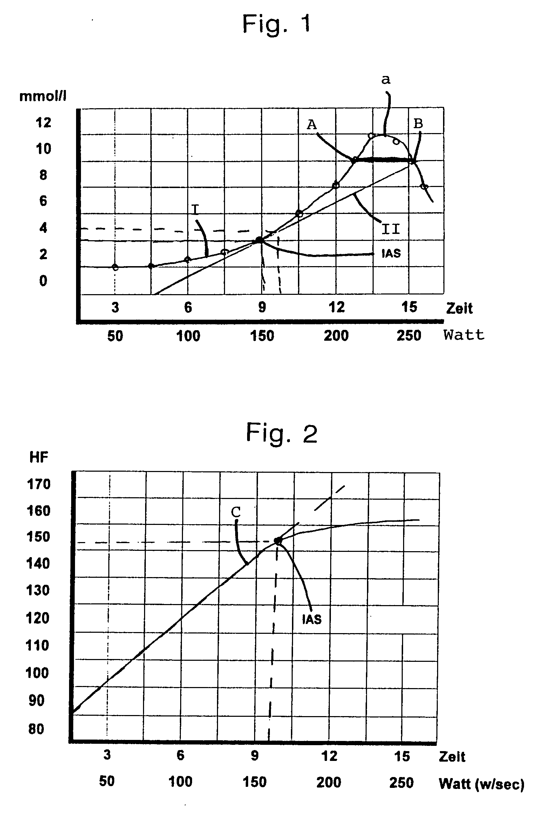

Gemäß Fig. 1 wird die Laktatkonstellation eines Trainierenden bei Belastung an einem Trainingsgerät zu vorgegebenen Zeiten gemessen und die jeweils erfaßten Meßwerte der Laktatkonstellation gemäß der Kurve I erfaßt und insbesondere in einen Rechner eingegeben, mittels welchem die Laktatleistungskurve I erfaßt bzw. ermittelt wird. An dem Punkt A wird die Belastung eingestellt und während der nachfolgenden Erholungsphase ergibt sich zunächst ein kurzer Anstieg der Laktatkonzentration mit nachfolgendem Abfall gemäß des Kurvenastes a bis gemäß Punkt B der Endbelastungslaktatwert A wieder erreicht ist. Der Punkt B wird als Erholungslaktatwert bezeichnet. Vom Erholungslaktatwert B wird nunmehr die Tangente II an die Laktatleistungskurve I gelegt. Der Berührungspunkt der Tangente II entspricht der individuellen anaeroben Laktatschwelle IAS. Wie ersichtlich, liegt die derart erfaßte individuelle anaerobene Laktatschwelle IAS des Trainierenden, dessen Laktatleistungskurve I erfaßt wurde, etwas niedriger als der oben erläuterte Wert von 4 mmol Laktat pro Liter Blut. Es sei festgehalten, daß bei der erläuterten erfindungsgemäß angewendeten Bestimmung der IAS die Laktatwerte in der unmittelbaren Nachbelastungsphase gemäß des Kurvenastes a mit berücksichtigt werden.

Es sei ferner darauf hingewiesen, dass die anaerobe Schwelle bei einer fixen Laktatkonzentration von 4 mmol/ Liter Blut von der Belastungsdauer je Belastungsstufe abhängig ist. Es wurde erkannt, dass mit zunehmender Dauer der Belastungsstufen die Leistungsfähigkeit im Bereich der anaeroben Schwelle abnimmt. Darüber hinaus wurde festgestellt, dass bei der Bestimmung der individuellen anaeroben Schwelle IAS in der vorstehend erläuterten Weise die genannte Abhängigkeit von der Dauer der Belastungsstufen nicht besteht. Wird die Belastung beispielsweise mittels einer Fahrradergometrie mit jeweils 5 Minuten dauernden Belastungsstufen durchgeführt, so lag bei Vorgabe der Schwelle von 4 mmol Laktat pro Liter Blut die Wattzahl deutlich niedriger als bei Belastungsstufen von jeweils 2 Minuten Dauer. Hingegen war die nach dem vorgenannten Verfahren ermittelte individuelle anaerobe Schwelle IAS in beiden Belastungsverfahren identisch. Die durchgeführten Untersuchungen ergaben, dass mit Ausnahme von höher Ausdauer trainierten Personen die anaerobe Schwelle im Mittel bei einer Laktatkonzentration von 4 mmol/l liegt. Da im Einzelfall aber erhebliche Abweichungen von diesem Mittelwert sich ergeben, wird in bevorzugter Weise in der Leistungsdiagnostik im Hochleistungssport und den daraus abgeleiteten Konsequenzen die Bestimmung der individuellen anaeroben Schwelle IAS vorgenommen.

Wie in Fig. 2 dargestellt, kann die Bestimmung der IAS zusätzlich oder alternativ derart durchgeführt werden, dass die Herzfrequenz HF bei Belastung in Abhängigkeit der Zeit erfasst wird und ferner die Leistung in Watt zugeordnet wird. Wie ersichtlich, nimmt am Anfang der Belastung an einem Trainingsgerät die Herzfrequenz linear zu und nach einer von der individuellen Leistungsfähigkeit abhängigen Zeitdauer, hier nach ca. 9,5 Minuten, ergibt sich an dem Punkt C eine deutliche Abflachung der Belastungskurve bzw. der Herzfrequenz. Dies ist ein Maß für die individuelle anaerobe Laktatschwelle.

Mittels des vorgeschlagenen Verfahrens, welches als Programm eines Rechners realisiert ist, erfolgt die Unterstützung der Auswertung der Leistungsmessung anhand der Bestimmung des Blutlaktatwertes. Es wird eine graphische Aufbereitung der Meßwerte und Auswertung der aktuellen Leistungsdaten durchgeführt. Darüber hinaus wird optional eine Trainingsempfehlung bei der Analyse erstellt.

Hierbei wird als Wert X die Leistung berücksichtigt, und zwar in Watt bezogen auf das Körpergewicht in Kilogramm gemäss folgender Bedingung:

$$X = \frac{Watt}{kg\ (K\ddot{o}rpergewicht)}$$

Ferner wird die Ist-Leistung Ni, und zwar gemessen in Watt, bei der vorgenannten Schwelle von insbesondere 4 mmol erfasst bzw. gelesen.

Weiterhin wird die Soll-Leistung Ns, und zwar gemessen in Watt, und unterschiedlich für Frauen und Männer unter Berücksichtigung des Gewichtes G und des Alters A nach folgender Bedingung bestimmt:

Frauen:      $Ns = f \times G\ (kg) \times [124 - 0,8 \times A\ (Jahre)\,] / 100$

Männer:      $Ns = m \times G\ (kg) \times [130 - 0,8 \times A\ (Jahre)] / 100$

Hierbei gelten für f Werte von 1 bis 2,5 und für m Werte von 1 bis 3.

Für die maximale Soll-Leistung $Ns_{max}$, gemessen in Watt/kg, wird die Bedingung berücksichtigt:

Frauen:      $Ns_{max} = 0,8\,[0,81 - 0,017 \times G\ (kg)]$

Männer:      $Ns_{max} = 7,81 - 0,027 \times G\ (kg)$

Durch die Festlegung der durchschnittlichen Fitness kann bedarfsweise die individuelle anaerobe Laktatschwelle IAS erhöht oder erniedrigt werden. Ferner sei festgehalten, dass ausschließlich ein Vergleichsparameter der Messung bzw. Messungen für eine bestimmte Gruppe von Menschen als konstant vorgegeben wird.

Im Rahmen der Erfindung liegen auch solche Modifikationen, gemäss welchen die in den vorgenannten Bedingungen enthaltenen Konstanten und / oder variable Parameter und/oder dementsprechend bestimmten Werte um bis zu ± 20 %, vorteilhaft bis zu ± 10 %, abweichen.

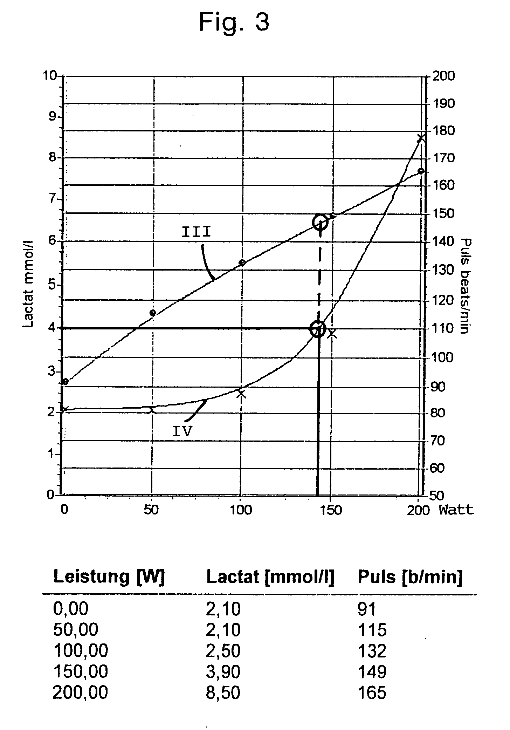

Weiterhin werden unter Berücksichtigung eines Diagramms vorgegebene Messwerte eingegeben, und zwar insbesondere betreffend das gebildete Laktat, die Herzfrequenz, die Leistung und die Zeit. In Fig. 3 ist ein derartiges typisches Diagramm dargestellt, in welchem die Kurve III die Herzfrequenz bzw. Pulsschläge pro Minute und die Kurve IV der Laktatkonzentration in mmol/l entsprechen. Unter dem Diagramm sind die während des Belastungstrainings eines Trainierenden erfaßten Meßwerte angegeben. Beim Überschreiten der aeroben / anaeroben Schwelle von 4 mmol/l Blut betrug die aktuelle Ist-Leistung 142,81 W. Hierbei betrug die Herz-Pulsfrequenz 146 Schläge pro Minute.

Unter Berücksichtigung der derart ermittelten Werte werden im Rahmen der Erfindung Vorschläge zur Trainingssteuerung berechnet bzw. erstellt. Die Trainingssteuerung umfaßt drei Zielsetzungen:

1. Eine langfristige Trainingssteuerung, die auf prozentualen Differenzen zwischen vorgebbaren Optimalwerten einerseits und Ist-Werten andererseits den verschiedenen Komponenten der aeroben und anaeroben Kapazität des Trainierenden beruht und zugleich auf die Priorität der zu verbessernden Komponenten hinweist.

2. Es erfolgt eine insbesondere tägliche Aufgabe der Trainingssteuerung, und zwar die Ermittlung der Bereitschaft des jeweiligen Trainierenden für das vorgesehene Training anhand des Verhältnisses aus Belastbarkeit zur Belastung.

3. Als letzte Zielsetzung der Trainingssteuerung folgt die Steuerung der Belastungsintensität während der jeweiligen Trainingseinheit. Hierzu dienen als Angaben insbesondere die Herzfrequenzwerte und/oder die m/s Werte und/oder die Zeit/Strecke Werte, welche unterschiedlichen Belastungsschwellen entsprechen.

Ausgehend von der Intensität der ermittelten Schwelle sind Intensitätsbereiche für das Training definiert. So entsprechen beispielsweise 95 bis 100% der Schwellenintensität einem intensiven Dauerlauftraining. Kleiner als 80% sind zweckmäßig für einen Regenerationslauf gewählt. Dazwischen liegt der Bereich eines extensiven Dauerlaufs und für ein Tempolauftraining werden bevorzugt Intensitätsvorgaben größer 100% gegeben.

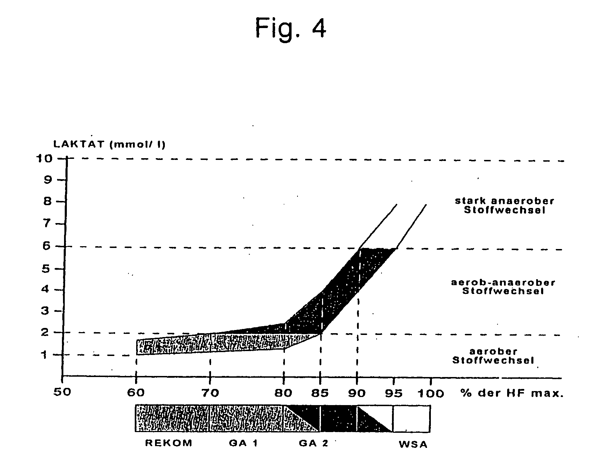

Fig. 4 zeigt ein bevorzugtes Diagramm der Trainingsbereiche, und zwar in Abhängigkeit der Laktatkonzentration und der Herzfrequenz. Hierin bedeuten REKOM ein Regenerations- und Kompensationstraining, GA ein Grundlagenausdauertraining, unterschieden in zwei Bereiche, GA1 für den Fettstoffwechsel und GA2 für Herz-Kreislauf und ferner WSA ein Wettkampf spezifisches Ausdauertraining.

Die nachfolgende Tabelle enthält einen Vorschlag der Trainingssteuerung für einen Trainierenden, dessen individuellen momentane Belastbarkeitsdaten in einem Leistungscheck mit den Werten gemäß Fig. 3 erfaßt wurden. Diese Daten wurden mit den weiteren persönlichen Daten, wie insbesondere Alter, Größe, Körpergewicht und Körperfettanteil in Prozent in dem gemäß des erfindungsgemäßen Verfahrens arbeitenden Rechner bzw. dessen Programm eingegeben zur Ermittlung des Vorschlags zur Trainingssteuerung.

|  | Fahrrad / Walking | Crosstrainer | Jogging / Laufband | Rudern | Dauer |
|---|---|---|---|---|---|
| **REKOM** | 112 - 119 | 122 - 130 | 125 - 133 | 127 - 134 | 10-30 min |
| **GA 1** | 119 - 126 | 130 - 137 | 133 - 141 | 134 - 142 | 1:00-3:00 h |
| **GA 2** | 135 - 140 | 147 - 153 | 151 - 157 | 152 - 158 | 0:20-1:00 h |
| **Tempo** | 140 - 152 | 153 - 166 | 157 - 170 | 158 - 172 | 10-30 min |

Die erfindungsgemässe Trainingssteuerung wird in vorteilhafter Weise durch folgende Verfahrensschritte realisiert:

d. Erfassung der aeroben / anaeroben Schwelle, insbesondere bei 4 mmol/l.

e. Erfassung der individuellen anaeroben Schwelle. Hierbei wird während einer Erholungsphase, in welcher der Laktatwert der Endbelastung wieder erreicht wird, eine Tangente an die Laktatleistungskurve gelegt und der Belastungswert im Berührungspunkt ermittelt, und zwar insbesondere die IAS nach Stegmann.

f. Bestimmung der IAS über die Herzfrequenz.

g. Prozentuelle Vorgabe je nach Trainingsziel.

**Patentansprüche**

1. Verfahren zur Trainingssteuerung, insbesondere zur Erlangung eines vorgebbaren Fettabbaus und / oder Muskelaufbaus und/oder Steigerung der Ausdauer oder Leistung einer oder eines Trainierenden, **dadurch gekennzeichnet, dass** der Laktatgehalt des Blutes der oder des Trainierenden erfasst wird und unter Berücksichtigung einer vorgegebenen oder vorgebbaren aeroben / anaeroben Schwelle, vorzugsweise zwischen 2,3 - 6,8 mmol Laktat/l Blut, insbesondere in der Grössenordnung von 4 mmol Laktat / l Blut, das Trainingsziel vorgegeben wird.

2. Verfahren nach Anspruch 1, **dadurch gekennzeichnet, dass** die individuelle anaerobe Laktatschwelle IAS des oder der Trainierenden bestimmt wird.

3. Verfahren nach Anspruch 1 oder 2, **dadurch gekennzeichnet, dass** die Ist-Leistung Ni mittels eines Trainingsgerätes erfasst und zur Auswertung berücksichtigt wird.

4. Verfahren nach einem der Ansprüche 1 bis 3, **dadurch gekennzeichnet, dass** unter Berücksichtigung des Gewichtes (G) sowie des Alters (A) der oder des Trainierenden die Soll-Leistung Ns, gemessen in Watt, gemäss folgender Bedingung bestimmt und berücksichtigt wird:

$$\text{Frauen:} \quad Ns = f \times G \, (kg) \times [124 - 0{,}8 \times A \, (\text{Jahre})] / 100$$

$$\text{Männer:} \quad Ns = m \times G \, (kg) \times [130 - 0{,}8 \times A \, (\text{Jahre})] / 100$$

wobei für f Werte von 1 bis 2,5 und für m Werte von 1 bis 3 gelten.

5. Verfahren nach einem der Ansprüche 1 bis 4, **dadurch gekennzeichnet, dass** die maximale Soll-Leistung Ns max' und zwar in Watt / Kilogramm, gemäss folgender Bedingung berücksichtigt wird:

$$\text{Frauen:} \quad Ns_{max} = 0{,}8 \, [0{,}81 - 0{,}017 \times G \, (kg)]$$

$$\text{Männer:} \quad Ns_{max} = 7{,}81 - 0{,}027 \times G \, (kg)$$

6. Verfahren nach einem der Ansprüche 1 bis 5, **dadurch gekennzeichnet, dass** die genannten Konstanten und / oder Parameter und / oder errechneten Werte bis zu $\pm$ 20 %, vorteilhaft bis zu $\pm$ 10 %, abweichen.

7. Verfahren nach einem der Ansprüche 1 bis 6, **dadurch gekennzeichnet, dass** folgende Verfahrensschritte durchgeführt und / oder Werte bevorzugt in Kombination wenigstens zwei derselben, insbesondere alle, berücksichtigt werden:

   a. Vorgabe und Berücksichtigung der aeroben / anaeroben Schwelle, insbesondere bei 4 mmol Laktat pro Liter Blut,
   b. Vorgabe und Berücksichtigung der individuellen anaeroben Schwelle IAS, wobei in einer Erholungsphase, in welcher der Endbelastungs-Laktatwert wieder erreicht ist eine Tangente an die Leistungskurve gelegt wird und der Belastungswert im Berührungspunkt erfasst wird.
   c. Vorgabe und Berücksichtigung der IAS über die Ermittlung der Herzfrequenz,
   d. prozentuelle Vorgabe je nach Trainingsziel.

8. Verfahren nach einem der Ansprüche 1 bis 7, **dadurch gekennzeichnet, dass** die Durchführung mittels eines Software-Programms und eines Rechners erfolgt.

Fig. 1

Fig. 2

# Fig. 3

| Leistung [W] | Lactat [mmol/l] | Puls [b/min] |
|---|---|---|
| 0,00 | 2,10 | 91 |
| 50,00 | 2,10 | 115 |
| 100,00 | 2,50 | 132 |
| 150,00 | 3,90 | 149 |
| 200,00 | 8,50 | 165 |

# Fig. 4